# EUROPEAN PATENT APPLICATION

(11) **EP 2 395 055 A1**
(43) Date of publication of application: **14.12.2011**
(21) Application number: 10738596.5
(22) Date of filing: 04.02.2010
(51) Int. Cl.: C09B 57/00, C07D 409/04, C09K 9/02, C09K 11/06, C12N 5/00, C12Q 1/02

(54) **FLUORESCENT SOLVATOCHROMIC PIGMENT**

(30) Priority: 06.02.2009 JP 2009026241
(71) Applicant: National University Corporation Hokkaido University, Sapporo-shi Hokkaido 060-0808 (JP)
(72) Inventor: YAMADA, Koji, Sapporo-shi, Hokkaido 060-0808 (JP); YAMAGISHI, Yutaka, Sapporo-shi, Hokkaido 060-0808 (JP); AYABE, Tokiyoshi, Sapporo-shi, Hokkaido 060-0808 (JP); SON, Sang-Hyun, Sapporo-shi, Hokkaido 060-0808 (JP); AOYAGI, Maiko, Sapporo-shi, Hokkaido 060-0808 (JP); TAIRA, Toshio, Sapporo-shi, Hokkaido 001-0021 (JP)
(74) Representative: Prüfer & Partner GbR European Patent Attorneys
(86) International application number: PCT/JP2010/051624
(87) International publication number: WO 2010/090265

(57) **Abstract**

The present invention presents a novel fluorescent solvatochromic dye that (1) has an ionic terminal that makes it easier to use n a hydrophilic surface or in polar solvents, (2) can be efficiently excited by commonly used Argon lasers (488 nm), (3) shifts the wavelength of emitted light according to the change of polarity, and (4) can effectively stain living tissues such as cells and the like.

A pyridinium group was introduced to the electron attracting group of the neutral fluorescent solvatochromic dye (Japanese Patent Application Public Disclosure No. 20008-291210 A) displaying an excellent emission wavelength response and synthesized a fluorescent solvatochromic dye. Then it was found that the fluorescence wavelength of the fluorescent solvatochromic dye changed extensively when the polarity changed on a hydrophilic surface and that the fluorescent solvatochromic dye, when altered to a cationic form, stained cell membranes and could be used to observe the behavior thereof.

## Description

### Field of Technology

The present invention relates to a fluorescent solvatochromic dye that undergoes a shift in its emitted light wavelength with solvent polarity, more specifically, to a fluorescent solvatochromic dye that can stain cells and undergoes a shift in its emitted light wavelength depending on the cell environment.

### Prior Art

Fluorescent solvatochromic dyes are dyes that change color depending on the polarity of the solvent surrounding the molecules and are currently used mainly as probes in high sensitivity real time observations of dynamics of biological molecules, particularly of lipid molecules, using fluorescence microscopes. The color changing mechanism thereof does not require contact with specific chemical species and can be used in identification of antigen antibody reactions and detection of monobasic polytypic species with high sensitivity, which are accomplished with difficulty when using other fluorescent dyes.
Such fluorescent solvatochromic dye includes NBD, Dansyl, Prodan, Dapoxyl and the like. Of these, Dapoxyl has the best performance and has an absorption band that can be excited by long-wavelength light (around 370 nm) which is unusual among solvatochromic dyes. Dapoxyl also has a significant Stoke's shift and a high fluorescence quantum yield (Reference 1).
The inventors previously developed a neutral fluorescent solvatochromic dye containing thiophene and the like as the base structure as means to shift the excitation wavelength to longer wavelengths and confirmed that the dye was efficiently excited using a blue diode laser (Reference 3).
In addition, commercially available DASPMI, a cationic fluorescent dye having a structure similar to the fluorescent solvatochromic dye of the present invention, is used mainly to observe mitochondria cell membrane behaviors (References 2 and 4).

### References

Reference 1: Photochemistry and Photobiology, 1997, 66(4): 424-43
Reference 2: Biochimica et Biophysica Acta, 1976, 423: 1-14
Reference 3: Japanese Patent Application Public Disclosure No. 2008-291210
Reference 4: Japanese Patent Application Public Disclosure No. 2003-506014

### Problems to be Solved by the Invention

Previously available fluorescent solvatochromic dyes (Reference 3) are highly soluble in fats, localize in hydrophobic cores of cell membranes and are used with difficulties on hydrophilic surfaces and in polar solvents. Furthermore, a fluorescent solvatochromic dye with the ability to be efficiently excited by a commonly used argon laser (488 nm), a light source emitting longer wavelength light than a blue diode laser (405 nm) and having no adverse effect on living tissues such as cells and the like, is desired.
In addition, the emission intensity of DASPMI (Reference 2) changes greatly in response to mitochondria activity, but the emission wavelength does not change. Therefore, DASPMI cannot be used in highly quantitative ratio-metric measurements and the like.
Therefore, the objective of the present invention is to present a novel fluorescent solvatochromic dye that (1) has an ionic terminal that makes it easier to use in a hydrophilic surface or in polar solvents, (2) can be efficiently excited by commonly used Argon lasers (488 nm), (3) shifts the wavelength of emitted light according to the change of polarity, and (4) can effectively stain living tissues such as cells and the like.

### Means to Solve the Problem

DASPMI (References 2 and 4), cationic fluorescent dyes used to observe mitochondria cell membrane behaviors, is represented by the following formula, and the pyridinium group thereof is a cationic substituent that is very strongly electron attractive. 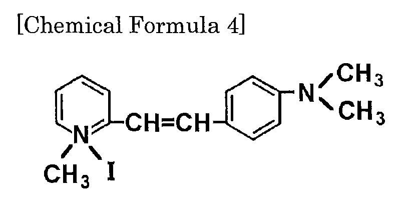 The pyridinium group is localized on the membrane surface in a mitochondria cell membrane, and the fluorescence intensity responds sensitively to the membrane potential.
Therefore, the inventors introduced a pyridinium group to the electron attracting group of the neutral fluorescent solvatochromic dye (Reference 3) displaying an excellent emission wavelength response and synthesized a fluorescent solvatochromic dye. The inventors discovered that the fluorescence wavelength of the fluorescent solvatochromic dye changed extensively when the polarity changed on a hydrophilic surface and that the fluorescent solvatochromic dye, when altered to a cationic form, stained cell membranes and could be used to observe the behavior thereof. The present invention was completed based on the discovery.

That is, the present invention is a fluorescent solvatochromic dye represented by the following formula. 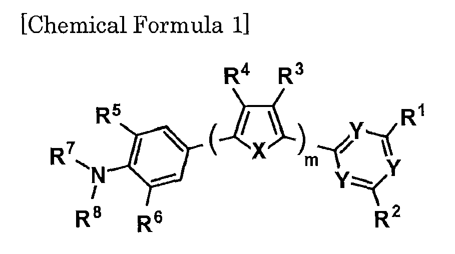 wherein,
X represents an oxygen atom (-O-) or a sulfur atom (-S-), m represents an integer from 1 to 4,
Y are independent each other and at least one of Y represents -N= with the remainder representing -CR⁹=,
R⁹ represents a hydrogen atom, an alkyl group with 1 to 4 carbon atoms or a group capable of bonding to other molecules,
R¹ and R² are independently hydrogen atoms, halogen atoms, primary or secondary amino groups or alkyl groups with 1 to 4 carbon atoms,
R³ and R⁴, which may be identical to or different from the other, are hydrogen atoms, alkoxy groups, acylamino groups, alkyl groups, halogen substituted alkyl groups, amino groups, hydroxyl groups or halogen atoms, and R³ and R⁴ may form, by sharing the carbon atoms bonding to R³ or R⁴, an aromatic or aliphatic 5, 6 or 8 membered ring that may contain ether bonds,
R⁵ and R⁶, which may be identical to or different from the other, are hydrogen atoms or alkyl groups,
R⁷ and R⁸, which may be identical to or different from the other, are hydrogen atoms or alkyl groups that may contain substituents, and R⁵ and R⁷ may form an aromatic or aliphatic 5 or 6 membered ring by sharing carbon atoms and nitrogen atoms bonding to R⁷ or R⁸, and R⁶ and R⁸ may form an aromatic or aliphatic 5 or 6 membered ring by sharing carbon atoms and nitrogen atoms bonding to R⁶ or R⁸.

In addition, the present invention is a cation type fluorescent solvatochromic dye obtained by introducing a cation into the fluorescent solvatochromic dye and represented by the formula above, wherein Y are independent each other and at least one of Y represents -N⁽⁺⁾R¹⁰=Z⁽⁻⁾ with the remainder representing -CR⁹=, wherein R⁹ is similarly defined as described above, R¹⁰ represents a hydrogen atom, a hydrocarbon group having 1 to 4 carbon atoms, or a group capable of bonding with other molecules, and Z represents a monovalent anionic species.
This cation type fluorescent solvatochromic dyes can be utilized as stains for cells, particularly for cell membranes. Therefore, the present invention is a cell staining agent comprising the cation type fluorescent solvatochromic dye. Furthermore, the present invention is a cell staining solution comprising the fluorescent solvatochromic dye dissolved or dispersed in an aqueous solvent. In addition, the present invention is a cell staining solution comprising the fluorescent solvatochromic dye dissolved or dispersed in serum. In addition, the present invention is a cell stained with the cation type fluorescent solvatochromic dye.

### Advantages of the Invention

The compound of the present invention is a novel fluorescent solvatochromic dye displaying a fluorescent solvatochromic nature in which the emission wavelength shifts with the solvent polarity.
The fluorescent solvatochromic dye is also useful as the intermediate used to synthesize a cation type fluorescent solvatochromic dye of the present invention.
The cation type fluorescent solvatochromic dye of the present invention is useful as the stain used to observe cells since the dye stains cells, particularly cell membrane, and the emission wavelength shifts according to the cell environment.
Furthermore, the nitrogen containing heterocyclic ring, such as a pyridinium group, that is the electron attracting group of the cation type fluorescent solvatochromic dye of the present invention, is ionic in nature, and the dye can be dissolved readily in a polar solvent such as an alcohol and the like by introducing a polar group such as a hydroxyl group and the like on the electron donating group section.

### Brief Description of the Drawings

Figure 1 shows the synthetic routes for N,N-dihexyl-4-[5-(pyriden-4-yl) thiophene-2-yl] aniline (Compound 3), N,N-dihexyl-4-[5-(pyridine-2-yl) thiophene-2-yl] aniline (Compound 4) and N,N-dihexyl-4-[5-(pyrimidine-2-yl) thiophene-2-yl] aniline (Compound 5) synthesized in Synthesis Example 1. The numbers in the figure refer to the compound numbers.

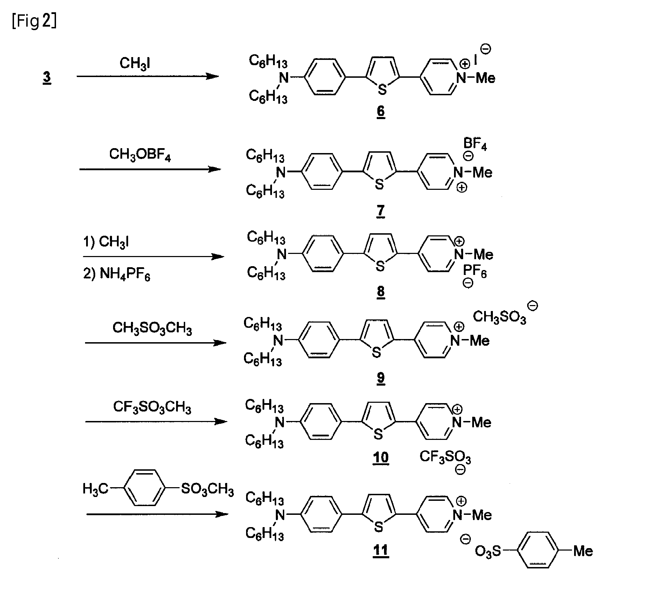

Figure 2 shows the synthetic routes for 4-{5-[4-(dihexylamino) phenyl] thiophene-2-yl} 1-methyl pyridinium iodide (Compound 6), 4-{5-[4-(dihexylamino) phenyl] thiophene-2-yl} 1-methyl pyridinium tetra-fluoroborate (Compound 7), 4-{5-[(4-(dihexylamino) phenyl] thiophene-2-yl} 1-methyl pyridinium hexafluorophosphate (Compound 8), 4-{5-[4-(dihexylamino) phenyl] thiophene-2-yl} 1-methyl pyridinium trifluoromethane sulfonate (Compound 9), 4-{5-[4-(dihexylamino) phenyl] thiophene-2-yl} 1-methyl pyridinium methane sulfonate (Compound 10) and 4-{5-[4-(dihexylamino) phenyl] thiophene-2-yl} 1-methyl pyridinium 4-methylbenzene sulfonate (Compound 11) synthesized in Synthesis Example 2. The numbers in the figure refer to the compound numbers.
Figure 3 shows the synthetic routes for 4-{5-[4-(dihexylamino) phenyl] thiophene-2-yl}-1-(2,5,8,11,14-pentaoxahexadecane-16-yl) pyridinium iodide (Compound 12) and 1-allyl-4-{5-[4-(dihexylamino)phenyl] thiophene-2-yl} pyridinium iodide (Compound 13) synthesized in Synthesis Example 3. The numbers in the figure refer to the compound numbers.
Figure 4 shows the synthetic route for 1-allyl-4-{5-[4-bis(2-hydroxyethyl) amino) phenyl] thiophene-2-yl} pyridinium iodide (Compound 15) synthesized in Synthesis Example 4. The numbers in the figure refer to the compound numbers.
Figure 5 shows the fluorescent emission spectrum of Compound 10 measured in the presence of additives in Example 3.
Figure 6 shows the fluorescent emission spectrum of Compound 24 measured in the presence of additives in Example 4.
Figure 7 is a fluorescent microscope image obtained by staining cultured human colon cancer cells T84 using Compound 7 in Example 3. The emitted color changes with location in the original color photograph, although the color change is not clearly seen in the present black and white photo.
Figure 8 (right) shows fluorescent spectra of segments shown in Figure 7. The figure on the left is the same as Figure 7, and the numbers in the figure indicate measurement locations.

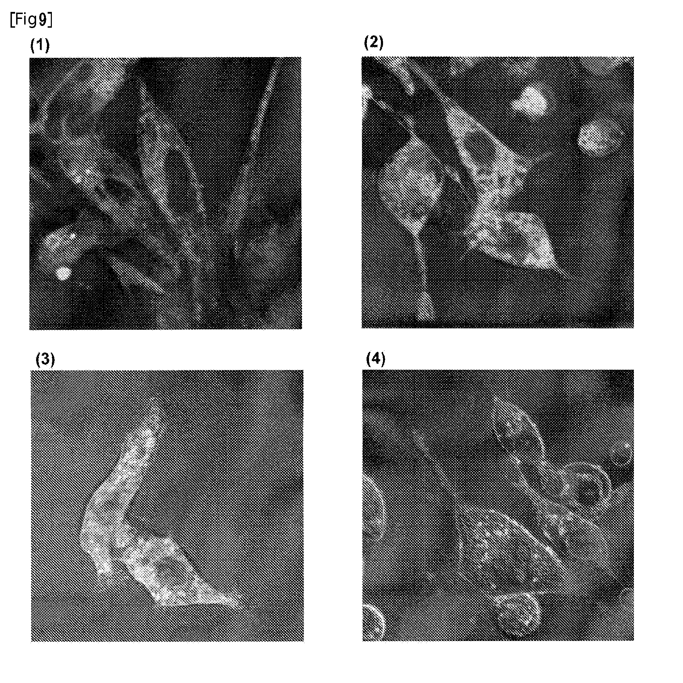

Figure 9 shows fluorescent microscope images of microphage cells stained with a variety of dyes. (1) shows the case in which Compound 21 is used as a dye, wherein R⁵ and R⁶ of chemical formula 1 are alkyl groups containing 2 carbon atoms. (2) shows the case in which Compound 22 is used as a dye, wherein R⁵ and R⁶ in chemical formula 1 are alkyl groups containing 4 carbon atoms. (3) shows the case in which Compound 10 is used as a dye, wherein R⁵ and R⁶ in chemical formula 1 are alkyl groups containing 6 carbon atoms. (4) shows the case in which Compound 23 is used as a dye , wherein R⁵ and R⁶ in chemical formula 1 are alkyl groups containing 6 carbon atoms.
Figure 10 shows the fluorescent microscope images of multiple numbers of different stained cells and the fluorescent spectra of the sections. The numbers in the figure indicate measurement locations, and "1" indicates a cell membrane, and "2" indicates a mitochondrion.
Figure 11 shows the fluorescent microscope image stained using the neutral type fluorescent solvatochromic dye of Comparative Example 1, and the fluorescent spectra of the sections. The numbers in the figure indicate the measurement locations.

### Detailed Description of the Invention

The fluorescent solvatochromic dye of the present invention (henceforth sometimes simply referred to as "the dye") is represented by the formula below. 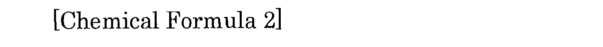 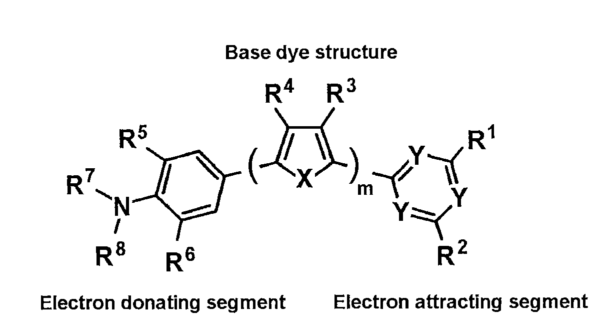 In the formula, X represents an oxygen atom (-O-) or a sulfur atom (-S-).
A thiophene derivative or a furan derivative containing sulfonic acid as the electron attracting segment can be synthesized readily by preparing a thiophene structure or a furan structure as the base dye structure and using the Suzuki-Miyaura cross coupling method to connect the electron donating segment and the electron attracting segment. In addition, multiple numbers of thiophene structures or furan structures may also be present as the dye base structure, and m represents an integer from 1 to 4, preferably 1 or 2.

Y are independent, and at least one of them (Y) represents -N= with the remainder representing -CR⁹=.
R⁹ represents a hydrogen atom, an alkyl group with 1 to 4 carbon atoms or a group capable of bonding to other molecules,
As the alkyl group, methyl, ethyl, propyl and butyl groups may be cited.
As the group capable of bonding to other molecules, halogen atom (fluorine atom, chlorine atom, bromine atom and iodine atom), amino group, hydroxyl group, carboxyl group, aldehyde group, vinyl group, ethenyl group, sulfonic acid group, maleimide group or alkyl group containing the group cited as a terminal may be cited. The alkyl group is preferably an alkyl group containing from 1 to 20 carbon atoms and is more preferably a linear group.

R¹ and R² each independently are hydrogen atom, halogen atom, primary or secondary amino group or alkyl group containing from 1 to 4 carbon atoms, preferably hydrogen atom, methyl group, chlorine atom or fluorine atom, more preferably hydrogen atom.

R³and R⁴, which may be identical to or different from the other, are hydrogen atom, alkoxy group, acylamino group, alkyl group, halogen substituted alkyl group, amino group, hydroxyl group or halogen group. The number of carbon atoms in the alkoxy group, acylamino group and alkyl group individually is preferably from 1 to 20, more preferably from 1 to 4. In addition, a chlorine atom or a fluorine atom is a preferred halogen atom.
Electron donating groups are preferred as the R³ and R⁴. As such electron donating group, alkoxy group, acylamino group, alkyl group, amino group or hydroxyl group, for example, may be cited and alkoxy group, alkyl group or hydroxyl group is preferably cited.
In addition, R³ and R⁴ may form, by sharing the carbon atoms bonding to R³ or R⁴, an aromatic or aliphatic 5, 6 or 8 membered ring that may contain ether bonds. For example, R³and R⁴ on the same thiophene ring or furan ring may jointly form an aromatic or aliphatic 5 or 6-membered ring that may contain ether (-O-) bond. Furthermore, when m is 2 or greater, the R³ or R⁴ on a thiophene ring or furan ring may form a 5, 6 or 8 membered ring, that may also contain an aromatic or ether (-O-) bond, jointly with the R³ or R⁴ on the adjacent thiophene ring or furan ring.

R⁵ and R⁶, which may be identical to or different from the other, are hydrogen atom or alkyl group. The alkyl group is preferably alkyl group containing from 1 to 4 carbon atoms.
R⁷ and R⁸, which may be identical to or different from the other, are hydrogen atom or alkyl group that may also contain substituents. The alkyl group is preferably alkyl group containing from 3 to 7 carbon atoms and is more preferably linear alkyl group, which is alkyl group represented by -CₙH₂ₙ₊₁ with n being 3 to 7, with structures similar to the lipids that are building blocks of the cell membrane. n is preferably, for example, 3 to 7. As the substituents, hydroxyl group, amino group, thiol group, sulfone group and the like may be cited.
In addition, R⁵ and R⁷ may form an aromatic or aliphatic 5 or 6 membered ring containing a nitrogen atom jointly with the carbon atom and nitrogen atom individually bonded to R⁵ and R⁷. R⁶ and R⁸ may form an aromatic or aliphatic 5 or 6 membered ring containing a nitrogen atom jointly with the carbon atom and nitrogen atom bonded to R⁶ and R⁸_{.}

An anion (Z·) may be further added to the dye of the present invention to form a cation type fluorescent solvatochromic dye. The anion is bonded by converting the nitrogen atom in the electron attracting segment of the dye into a cation, and three locations from the nitrogen atom sites may be converted into cations. In this case, at least one of Y in the chemical formula 1 described above represents -N^{(+)R}R¹⁰=Z^{(·)} and the remainder represents -CR⁹=.
R¹⁰ represents a hydrogen atom, hydrocarbon group, a group capable of bonding to other molecules, or a hydrocarbon group bonded to a group capable of bonding to other molecules and preferably represents a hydrogen atom or a hydrocarbon group. The hydrocarbon group is preferably an alkyl group and is more preferably an alkyl group containing from 1 to 4 carbon atoms. As a group capable of bonding to other molecules, a vinyl group, epoxy group, amino group, hydroxyl group, carboxyl group, aldehyde group, isocyanate group, isothiocyanate group, phosphoric acid group, thiol group, maleimide group, N-hydroxy succinimide group, vinyl sulfone group and the like may be cited.
The some structures of a cation type dye wherein a nitrogen atom becomes the cation are shown in the formulas below. Of these, the dye with the structure (1) is preferred since the wavelength at the maximum absorption is near the wavelength of an argon laser and the fluorescent intensity is high.

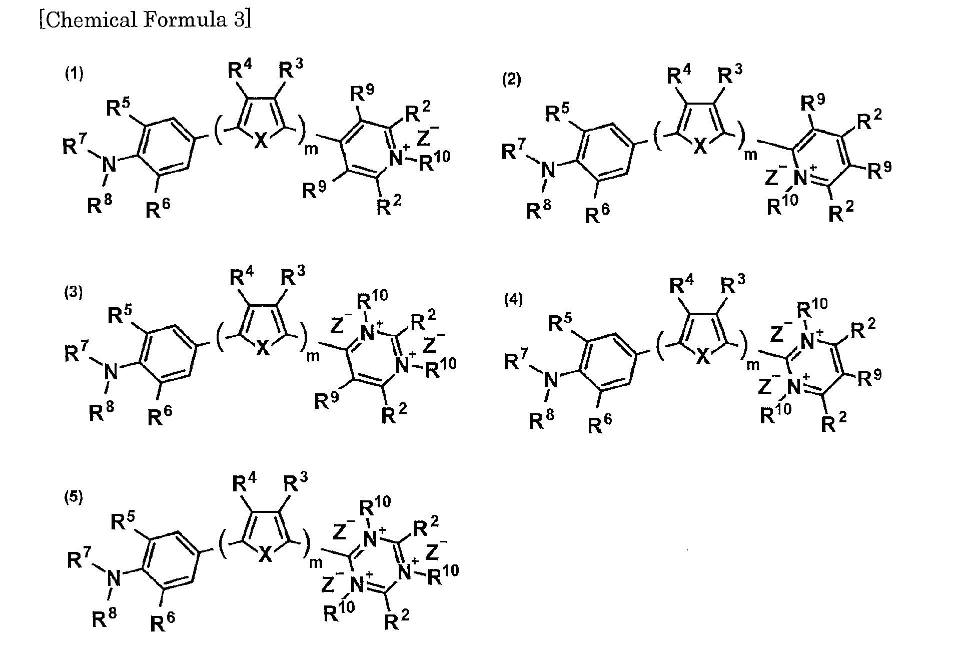

Z represents a monovalent anionic species. As the monovalent anionic species, fluoride anion, chloride anion, bromide anion, iodide anion, tetrafluoroborate anion, hexafluorophosphate anion, acetic acid anion, trifluoroacetic acid anion, sulfate anion, hydrogen sulfate anion, methane sulfate anion, trifluoromethane sulfate anion, perchlorate anion, hexachloroantimonate anion, bis(trifluoromethane sulfonyl) imide anion, and N-trifluoromethane sulfonyl-N-trifluoromethane acetyl imide anion may be cited.
As a method used to introduce a cation, a method in which a compound represented by either the chemical formula 1 or 2, wherein at least one of Y represents-N= with the remainder representing -CR⁹=, and a compound comprising a monovalent anion species (for example, bromine, iodine, trifluoromethane sulfate) are allowed to react at a temperature between 0°C and the reflux temperature of the solvent may be cited. In addition, the anion, which is Z in the chemical formula 1 or 2, of the compound obtained by once introducing a cation can be exchanged to an optional other monovalent anion using an anionic exchange resin.

Such cation type dyes can be readily dissolved or dispersed in an organic solvent or an aqueous medium. By using the dye in the form of a stain solution obtained by dissolving or dispersing a dye in an organic solvent or an aqueous medium, cells cultured in a culture solution and the like can be stained.
The cation type dye is readily dissolved in an organic solvent. However, the cation type dye is difficult to dissolve in pure water. Therefore, aqueous media are used. More preferably, said cation type dye can be dissolved or dispersed in an aqueous solvent by having a substance with an emulsification action or a substance with an encapsulating capability present in the aqueous solvent.
As such organic solvents, hydrocarbon type solvents such as toluene and the like, halogenated solvents such as methylene chloride, chloroform and the like, ether type solvents such as 1,4-dioxane, tetrahydrofuran and the like, ester type solvents such as ethyl acetate and the like, ketone type solvents such as acetone and the like, dimethyl formamide, dimethyl sulfoxide and the like may be used.
As the aqueous media, mixed solutions of water and alcohol or acetonitrile can be cited, but various cell culture solutions, serums and the like may also be used.
As the substance with an emulsifying action, surfactants, lipids, saponins, peptides, choleric acid and the like may be cited. As the substance with an encapsulating capability, crown ethers, cyclodextrins, calixarenes, nucleic acids and the like can be cited. The concentration of such a substance in a solvent is ordinarily about from 10⁻⁵M to 10⁻²M.
The use of a staining solution that is a dispersion or solution in an aqueous medium is preferred since an organic medium alone is sometimes toxic to cells.

Such a dye molecule described above may be used in, for example, the following applications since it can detect polarity changes in the environment surrounding the dye.
(1) The dye molecule of the present invention is useful as a probe for living membranes in the field of fluorescent dyes used in bio-imaging. That is, such a molecule can be utilized in a molecular membrane probe that changes its fluorescent color in response to a localized environment by connecting the molecule to alkyl chains and the like of lipid molecules. A cation type and ampho-ion type living membrane probe can be obtained by introducing a fat soluble substituent such as a long chain alkyl group and the like to the R⁷ or R⁸ of the compound represented by the chemical formula 1 or 2 and introducing an alkyl group or an alkyl group containing an acidic terminal such as sulfonic acid and the like to the Y in the chemical formula 1 or 2. The molecule emits long wavelength fluorescence in a hydrophilic environment and short wavelength fluorescence in a hydrophobic environment. Therefore, the molecule can be used for high sensitivity real time measurements of living membrane dynamics such as evaluation and detection of antibacterial action in a bacterial cell membrane and evaluation of a drug delivery system.

(2) The molecule of the present invention is useful in high sensitivity detection of antigen-antibody reactions in the immuno-assay field. That is, the molecule can be utilized as a probe that can measure antigen-antibody reactions with high sensitivity and quantitative precision when used to label a specific amino acid residue of an antigen or an antibody. The probe is, for example, a compound obtained by introducing an alkyl group containing a terminal maleimide group to the Y in the chemical formula 1 or 2. When the labeling occurs in a specific amino acid segment in the vicinity of the antigen/antibody recognition segment, long wavelength fluorescence is thought to be emitted when the segment is exposed to a protein surface prior to the reaction and short wavelength fluorescence is thought to be emitted when the segment is incorporated into a hydrophobic site after the reaction. The fluorescent solvatochromic dye of the present invention does not require recognition of a specific substituent and can be generically used in a variety of antigen antibody reactions. In addition, the fluorescent solvatochromic dye emits fluorescence at multiple wavelengths, and the antigen-antibody reaction can be detected highly quantitatively by calculating the intensity ratios of the wavelengths.

(3) The molecule of the present invention can be used in single nucleotide polymorphism detections as a probe to identify a specific nucleotide sequence with high sensitivity by emitting a specific fluorescence color. For example, a specific nucleotide can be labeled with fluorescence when a pyridine starting material of the dye is connected to a specific nucleotide in a nucleotide sequence through a suitable spacer (for example, methylene group) by using a pyridine derivative containing a terminal halogen atom. A nucleic acid amidite is synthesized using a previously known method, and a nucleic acid polymer incorporating a desired nucleotide sequence can be synthesized using a solid phase synthesis process. The hybridization of a completely complementary nucleic acid polymer to the nucleotide sequence forms a hydrophobic site. Therefore the fluorescence from the dye is short wavelength. But the fluorescence from the dye becomes longer wavelength, when a base pair mismatch is present in the vicinity and hydrophilicity is increased. Therefore, the nucleotide sequence can be identified with high sensitivity using the fluorescence color.
Since the dye of the present invention has an absorption wavelength in the long wavelength region, damages to protein and DNA caused by the excitation light can be avoided. Therefore, the dye of the present invention has more advantageous than previously known dyes in these applications.

(4) The dye of the present invention can stain cells, which allows the cell condition to be studied. For example, differentiation of stem cell can be traced. In addition, different cells can be identified. The cell that can be stained by the dye of the present invention includes, not particularly limited, for example, procariotic cells (bacteria), plant cells, reproductive cells, somatic cells (stem cells, fat cells, liver cells, white corpuscles, red corpuscles, platelets), cancer cells and the like.

### Examples

The following Examples illustrate the present invention, but the Examples are not intended to limit the present invention.

### Synthesis Example 1

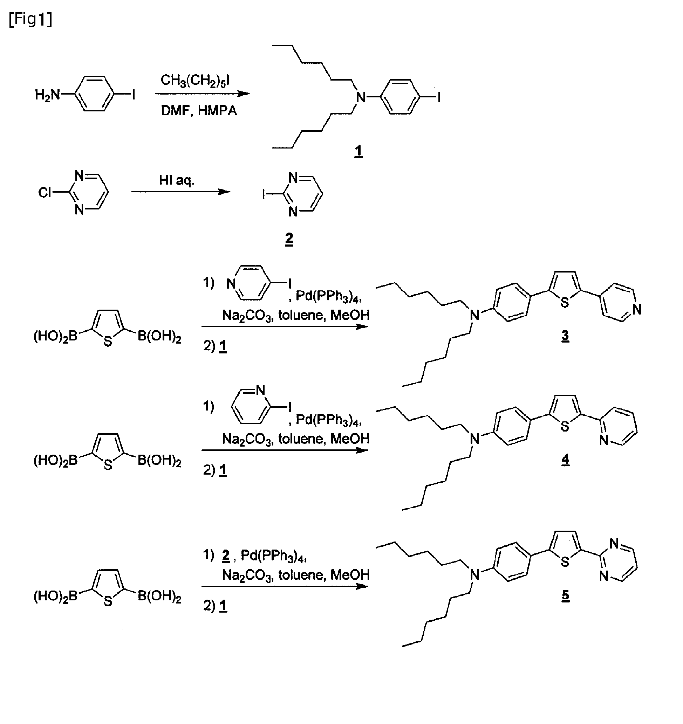

In the present synthesis example, N,N-dihexyl-4-[5-(pyridine-4-yl) thiophene-2-yl] aniline (Compound 3), N,N-dihexyl-4-[5-(pyridine-2-yl) thiophene-2-yl] aniline (Compound 4) and N,N-dihexyl-4-[5-(pyrimidine-2-yl) thiophene-2-yl] aniline (Compound 5) are prepared. The synthetic route is shown in Figure 1.
Potassium carbonate (7.56 g, 54.8 mmoles) was added to a solution obtained by dissolving 6.00 g (27.4 mmoles) of 4-iodoaniline (Tokyo Chemical Industry Co., Ltd.) and 17.4 g (82.2 mmoles) of 1-iodohexane (Sigma-Aldrich Corporation) in a mixed solvent of 17.9 ml of DMF and 9.50 ml of HMPA. The mixture was agitated for twenty-two hours at 90°C. The mixture was left standing to cool, ethyl acetate was added, and the white precipitate was removed by filtration. The filtrate was diluted using ethyl acetate and was washed twice using de-ionized water, once with saturated aqueous sodium bicarbonate solution and once using saturated sodium chloride solution. The organic layer was dried using anhydrous sodium sulfate. The solvent was removed, and the product was purified using a medium pressure fractionating liquid chromatograph to obtain 9.24 g (23.9 mmoles) of a colorless, clear oil (Compound 1). The analytical results (N,N-dihexyl-4-iodoaniline) of the Compound 1 obtained are shown below.
¹H-NMR (400 MHz, CDCl₃, TMS, rt) δ 7.41 (2H, d, J = 8.6 Hz), 6.40 (2H, d, J = 8.8 Hz), 3.21 (4H, t, J = 7.9 Hz), 1.54 (4H, brs), 1.31 (12H, brs), 0.90 (6H, t, J = 6.7 Hz)

Ice cooled hydriodic acid (Wako Pure Chemical Industries, Ltd.) (14.7 ml, 57%) was added gradually to 3.68 g (32.0 mmoles) of 2-chloropyrimidine (Sigma-Aldrich Corporation), and the reaction mixture was agitated for fifty minutes at 0°C. Ice cooled aqueous sodium carbonate solution was added to the reaction solution until the solution was neutral, and aqueous sodium sulfite solution was subsequently added. The product was extracted using diethyl ether, and the organic layer was dried using anhydrous sodium sulfate after it was washed once using a saturated aqueous sodium chloride solution. The solvent was removed, and the pale yellow oil remaining was dissolved in boiling hexane. The solution was left standing to cool, and 3.62 g (17.6 mmoles, 55%) of colorless needle-like crystals (Compound 2) was obtained. The analytical results for Compound 2 obtained (2-iodopyrimidene) are shown below.
¹H-NMR (400 MHz, CDCl₃, TMS, rt) δ 8.47 (2H, d, J = 4.8 Hz), 7.32 (1H, t, J = 4.9 Hz)

4-Bromopyridine hydrochloride salt (Wako Pure Chemical Industries, Ltd.) (1.67 g, 8.61 mmoles), 2,5-thiophene diborate (Wako Pure Chemical Industries, Ltd.) (4.44 g, 25.8 mmoles), sodium carbonate (Wako Pure Chemical Industries, Ltd.) (7.30 g, 68.9 mmoles) and tetrakis (triphenylphosphine) palladium (0) (Wako Pure Chemical Industries, Ltd.) (299 mg, 0.259 mmoles) were dissolved in a mixed solvent containing 75 ml of toluene and 25 ml of methanol. Air was removed to create a vacuum and nitrogen was substituted. The process was repeated three times, and the reaction mixture was agitated for thirty minutes at 70°C. After letting the reaction solution stand until cooled, Compound 1 obtained in the manner described above was added. The air was removed to create a vacuum and nitrogen was substituted. The process was repeated three times again, and the reaction mixture was agitated for nine hours at 70°C. After allowing the reaction solution to cool, the reaction solution was diluted with ethyl acetate, washed three times using de-ionized water, and once using an aqueous saturated sodium chloride solution. The organic layer was dried using anhydrous sodium sulfate. The solvent was removed by distillation, and the product was purified using medium pressure fractionating liquid chromatography to obtain a yellow powder (Compound 3) (1.09 g, 2.59 mmoles, 30%). The analytical results for the Compound 3 synthesized [N,N-dihexyl-4-(5-(pyridine-4-yl) thiophene-2-yl] aniline} are shown below. 1H-NMR (400 MHz, CDCl3, TMS, rt) δ 8.56-8.54 (2H, m), 7.49-7.43 (5H, m), 7.13 (1H, d, J = 3.8 Hz), 6.65-6.63 (2H, d, J = 9.0 Hz), 3.30 (4H, t, J = 7.7 Hz), 1.61 (4H, brs), 1.34 (12H, brs), 0.92 (6H, t, J = 6.8 Hz)

2-Iodopyridine (Tokyo Chemical Industry Co., Ltd.) (200 mg, 0.976 mmoles), 2,5-thiophene diborate (Wako Pure Chemical Industries, Ltd.) (420 mg, 2.44 mmoles), sodium carbonate (Wako Pure Chemical Industries, Ltd.) (311 mg, 2.93 mmoles) and tetrakis (triphenylphosphine) palladium (0) (Wako Pure Chemical Industries, Ltd.) (33.9 mg, 0.0293 mmoles) were dissolved in a mixed solvent containing 5 ml of toluene and 5 ml of methanol. Air was removed to create a vacuum and the space was purged with nitrogen three times after which the reaction mixture was agitated for thirty minutes at 80°C. After letting the reaction solution stand until cooled, Compound 1 obtained in the manner described above was added. The air was removed to create a vacuum and the space was purged with nitrogen three times again. after which the reaction mixture was agitated for seven hours at 80°C. After allowing the reaction solution to cool, the reaction solution was diluted with ethyl acetate, washed once using de-ionized water, and once using an aqueous saturated sodium chloride solution. The organic layer was dried using anhydrous sodium sulfate. The solvent was removed by distillation, and the product was purified using a medium pressure fractionating liquid chromatography to obtain yellow solids (Compound 4) (99.2 mg, 0.236 mmoles, 24%). The analytical results for the Compound 4 synthesized {N,N-dihexyl-4-[5-(pyridine-2-yl) thiophene-2-yl] aniline} are shown below.
¹H-NMR (400 MHz, CDCl₃, TMS, rt) δ 8.55 (1H, brd, J = 4.5 Hz), 7.67-7.61 (2H, m), 7.53-7.50 (3H, m), 7.14-7.08 (2H, m), 6.64 (2H, d, J = 8.6 Hz) 3.29 (4H, t, J = 7.5 Hz), 1.60 (4H, brs), 1.33 (12H, brs), 0.91 (6H, t, J = 6.3 Hz)

Compound 2 obtained in the manner described above (200 mg, 0.971 mmoles), 2,5-thiophene diborate (Wako Pure Chemical Industries, Ltd.) (501 mg, 2.91 mmoles), sodium carbonate (Wako Pure Chemical Industries, Ltd.) (309 mg, 2.91 mmoles) and tetrakis (triphenylphosphine) palladium (0) (Wako Pure Chemical Industries, Ltd.) (33.7 mg, 0.0292 mmoles) were dissolved in a mixed solvent containing 10 ml of toluene and 10 ml of methanol. Air was removed to create a vacuum and the space was purged with nitrogen three times after which the reaction mixture was agitated for thirty minutes at 60°C. After letting the reaction solution stand until cooled, Compound 1 obtained in the manner described above was added. The air was removed to create a vacuum and the space was purged with nitrogen three times again after which the reaction mixture was agitated for four hours at 60°C. After allowing the reaction solution to cool, the reaction solution was diluted with ethyl acetate, washed once using de-ionized water, and once using an aqueous saturated sodium chloride solution. The organic layer was dried using anhydrous sodium sulfate. The solvent was removed by distillation, and the product was purified using a medium pressure fractionating liquid chromatography to obtain yellow solids (Compound 5) (191 mg, 0.453 mmoles, 47%). The analytical results for the Compound 5 synthesized {N,N-dihexyl-4-[5-(pyrimidine-2-yl) thiophene-2-yl] aniline} are shown below.
¹H-NMR (400 MHz, CDCl₃, TMS, rt) δ 8.67 (2H, d, J = 4.9 Hz), 7.92 (1H, d, J = 3.9 Hz), 7.53 (2H, brd, J = 8.8 Hz), 7.18 (1H, d, J = 3.8 Hz), 7.03 (1H, t, J = 4.9 Hz), 6.64 (2H, brd, J = 8.8 Hz), 3.29 (4H, t, J = 7.7 Hz), 1.60 (4H, brs), 1.33 (12H, brs), 0.91 (6H, t, J = 6.5 Hz)

### Synthesis Example 2

In the present synthesis example, 4-[5-(4-dihexylamino) phenyl] thiophene-2-yl-1-methyl pyridinium iodide (Compound 6), 4-[5-(4-dihexylamino) phenyl] thiophene-2-yl-1-methyl pyridinium tetrafluoroborate (Compound 7), 4-[5-(4-dihexylamino) phenyl] thiophene-2-yl-1-methyl pyridinium hexafluorophosphate (Compound 8), 4-[5-(4-dihexylamino) phenyl] thiophene-2-yl-1-methyl pyridinium hexafluoromethane sulfonate (Compound 9), 4-[5-(4-dihexylamino) phenyl] thiophene-2-yl-1-methyl pyridinium methane sulfonate (Compound 10) and 4-[5-(4-dihexylamino) phenyl] thiophene-2-yl-1-methyl pyridinium 4-methylbenzene sulfonate (Compound 11) were synthesized. The synthesis route is shown in Figure 2.

Compound 3 (47.4 mg, 0.113 mmoles) obtained in Synthesis Example 1 was dissolved in 2.0 ml of methylene chloride, and 702 µl of methyl iodide (11.3 mmoles) (Kanto Chemical Co., Ltd.) was added. The reaction solution was heated and refluxed for 1.5 hours. The reaction solution was left to cool after which the solvent and methyl iodide were removed by distillation to yield dark red solids (Compound 6) (62.9 mg, 0.112 mmoles). The analytical results for the Compound 6 {4-[5-(4-dihexylamino) phenyl] thiophene-2-yl-1-methyl pyridinium iodide} obtained are shown below. ¹H-NMR (400 MHz, CDCl₃, TMS, rt) δ 8.85 (2H, d, J = 6.8 Hz), 7.88 (2H, d, J = 7.0 Hz), 7.80 (1H, d, J = 4.2 Hz), 7.50 (2H, d, J = 8.8 Hz), 7.25 (1H, d, J = 4.0 Hz), 6.63 (2H, d, J = 8.9 Hz), 4.47 (3H, s), 3.32 (4H, t, 7.8 Hz), 1.60 (4H, brs), 1.34 (12H, brs), 0.92 (6H, t, J = 6.7 Hz)

Compound 3 (100 mg, 0.238 mmoles) obtained in Synthesis Example 1 was dissolved in 4.0 ml of methylene chloride, and an ice cold methylene chloride solution (0.357 M) of tetrafluoroboric acid trimethyl oxonium (Wako Pure Chemical Industries, Ltd.) (52.8 mg, 0.357 mmoles) was gradually added. The reaction solution was agitated for an hour at 0°C and was diluted using methylene chloride. The solution was washed with de-ionized water and with saturated aqueous sodium chloride solution, and the organic layer was dried with anhydrous sodium sulfate. The solvent was removed by distillation, and the product was subsequently purified using medium pressure fractionating liquid chromatography to obtain dark red solids (Compound 7) (124 mg, 0.238 mmoles, and quantitative yield). The analytical results for the Compound 7 {4-[5-(4-dihexylamino) phenyl] thiophene-2-yl-1-methyl pyridinium tetrafluoroborate} obtained are shown below.
¹H-NMR (400 MHz, CDCl₃, TMS, rt) δ 8.83 (2H, d, J = 6.8 Hz), 7.82 (2H, d, J = 6.8 Hz), 7.76 (1H, d, J = 4.2 Hz), 7.48 (2H, d, J = 8.9 Hz), 7.23 (1H, d, J = 4.2 Hz), 6.62 (2H, d, J = 8.9 Hz), 4.31 (3H, s), 3.31 (4H, t, 7.7 Hz), 1.61 (4H, brs), 1.34 (12H, brs), 0.92 (6H, t, J = 6.6 Hz) ; HRMS (ESI) calculated. For C₂₈H₃₉N₂S [M]⁺ 435.2828, found 435.2834; MS (ESI) calculated. For BF₄ [M]· 87.0035, found 87.0162.

Compound 3 (30.3 mg, 0.0720 mmoles) obtained in Synthesis Example 1 was dissolved in 1.0 ml of methylene chloride, and 224 µl of methyl iodide (3.60 mmoles) (Kanto Chemical Co., Ltd.) was added. The reaction solution was heated and refluxed for two hours. The reaction solution was left to cool after which the solvent and methyl iodide were removed by distillation. The dark red solids obtained were dissolved in 1.0 ml of methylene chloride, 58.9 mg of ammonium hexafluorophosphate (Kanto Chemical Co., Ltd.) (0.360 mmoles) was added and the solution was agitated for forty-eight hours at room temperature. The reaction solution was diluted with methylene chloride, and the solution was washed with de-ionized water and with saturated aqueous sodium chloride solution. The organic layer was dried using anhydrous sodium sulfate. The solvent was removed by distillation, and the product was subsequently purified using medium pressure fractionating liquid chromatography to yield deep red solids (Compound 8) (41.8 mg, 0.0720 mmoles). The analytical results for the Compound 8 {4-[5-(4-dihexylamino) phenyl] thiophene-2-yl-1-methyl pyridinium hexafluorophosphate} obtained are shown below.
¹H-NMR (400 MHz, CDCl₃, TMS, rt) δ 8.81 (2H, d, J = 7.2 Hz), 7.81 (2H, d, J = 7.2 Hz), 7.76 (1H, d, J = 4.2 Hz), 7.50 (2H, d, J = 9.0 Hz), 7.24 (1H, d, J = 3.6 Hz), 6.63 (2H, d, J = 8.9 Hz), 4.24 (3H, s), 3.32 (4H, t, 7.7 Hz), 1.60 (4H, brs), 1.33 (12H, brs), 0.92 (6H, t, J = 7.0 Hz); HRMS (ESI) calculated. For C₂₈H₃₉N₂S [M]⁺ 435.2828, found 435.2834; MS (ESI) calculated. For F₆P [M]⁻ 144.9642, found 144.9544.

Compound 3 (50.2 mg, 0.119 mmoles) obtained in the Synthesis Example 1 was dissolved in 2.0 ml of methylene chloride, and 320 µl of methyl methane sulfonate (Sigma-Aldrich Corporation) (3.78 mmoles) was added in several portions. The reaction mixture was agitated at room temperature for thirty hours. The solvent was removed by distillation, and the product was purified using medium pressure fractionating liquid chromatography to yield dark red solids (Compound 9) (61.2 mg, 0.115 mmoles, 97%). The analytical results for the Compound 9 {4-[5-(4-dihexylamino) phenyl] thiophene-2-yl]-1-methyl pyridinium methane sulfonate} obtained are shown.
¹H-NMR (400 MHz, CDCl₃, TMS, rt) δ 8.82 (2H, d, J = 7.0 Hz), 7.83 (2H, d, J = 7.0 Hz), 7.72 (1H, d, J = 4.2 Hz), 7.38 (2H, d, J = 9.0 Hz), 7.10 (1H, d, J = 4.1 Hz), 6.55 (2H, d, J = 9.0 Hz), 4.32 (3H, s), 3.27 (4H, t, 7.7 Hz), 2.82 (3H, s), 1.58 (4H, brs), 1.32 (12H, brs), 0.91 (6H, t, J = 6.8 Hz)

Compound 3 (36.0 mg, 0.0855 mmoles) obtained in Synthesis Example 1 was dissolved in 2.0 ml of methylene chloride, and the solution was cooled with ice. Methyl trifluoromethane sulfonate (Tokyo Chemical Industry Co., Ltd.) (11.7 µl, 0.103 mmoles) was gradually added, and the reaction solution was agitated for an hour at 0°C. After the solvent was removed by distillation, the product was purified using medium pressure fractionating liquid chromatography to yield reddish purple solids (Compound 10) (50.0 mg, 0.0855 mmoles, quant). The analytical results for {4-[5-(4-dihexylamino) phenyl] thiophene-2-yl}-1-methyl pyridinium trifluoromethane sulfonate (Compound 10) are shown below.
¹H-NMR (400 MHz, CDCl₃, TMS, rt) δ 8.51 (2H, d, J = 7.0 Hz), 7.84 (2H, d, J = 7.2 Hz), 7.79 (1H, d, J = 4.2 Hz), 7.51 (2H, d, J = 8.9 Hz), 7.26 (1H, brs), 6.63 (2H, d, J = 9.0 Hz), 4.32 (3H, s), 3.32 (4H, t, 7.7 Hz), 1.60 (4H, brs), 1.34 (12H, brs), 0.92 (6H, t, J = 6.5 Hz); HRMS (ESI) calculated. For C₂₈H₃₉N₂S [M]⁺ 435.2828, found 435.2834; MS (ESI) calculated. For CF₃O₃S [M]⁻ 148.9526, found 148.9414.

Compound 3 (49.4 mg, 0.117 mmoles) obtained in Synthesis Example 1 was dissolved in 2.0 ml of methylene chloride, and the solution was cooled with ice. Methyl p-toluene sulfonate (Sigma-Aldrich Corporation) (371 µl, 2.45 mmoles) was added in several portions, and the reaction solution was agitated for fifty hours at room temperature. After the solvent was removed by distillation, the product was purified using medium pressure fractionating liquid chromatography to yield dark red solids (Compound 11) (33.8 mg, 0.0557 mmoles, 48%). The analytical results for {4-[5-(4-dihexylamino) phenyl] thiophene-2-yl}-1-methyl pyridinium 4-methylbenzene sulfonate (Compound 11) are shown below.
¹H-NMR (400 MHz, CDCl₃, TMS, rt) δ 8.77 (2H, d, J = 6.3 Hz), 7.82-7.78 (4H, m), 7.72 (1H, d, J = 3.8 Hz), 7.43 (2H, d, J = 8.4 Hz), 7.14-7.12 (3H, m), 6.59 (2H, d, J = 8.7 Hz), 4.35 (3H, s), 3.30 (4H, t, 7.5 Hz), 2.31 (3H, s), 1.60 (4H, brs), 1.34 (12H, brs), 0.92 (6H, m)

### Synthesis Example 3

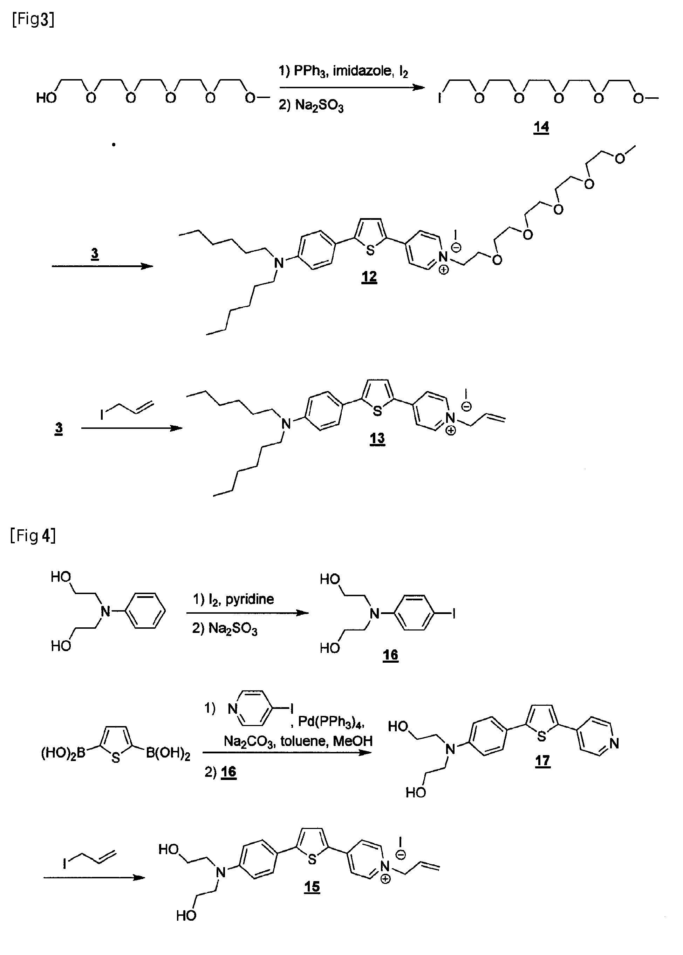

In the present synthesis example, 4-{5-[4-(dihexylamino)phenyl] thiophene-2-yl}-1-(2,5,8,11,14-pentaoxahexadecane-16-yl) pyridinium iodide (Compound 12) to which oligo-ethylene glycol segments that are effective in reducing cell toxicity had been introduced and 1-allyl-4-{5-[4-(dihexylamino) phenyl] thiophene-2-yl} pyridinium iodide (Compound 13) containing terminal polymer segments were synthesized. The synthesis route is shown in Figure 3.
Triphenyl phosphine (Wako Pure Chemical Industries, Ltd.) (624 mg, 2.38 mmoles) and 162 mg of imidazole (2.38 mmoles) were dissolved in 20.0 ml of methylene chloride, and the solution was cooled with ice. Iodine (Wako Pure Chemical Industries, Ltd.) (605 mg, 2.38 mmoles) was added, and the reaction solution was agitated for five minutes at 0°C. A methylene chloride solution (0.198 M) of 500 mg of pentaethylene glycol monomethyl ether (Tokyo Chemical Industry Co., Ltd.) (1.98 mmoles) was gradually added, and the solution was agitated for six hours at room temperature. An aqueous sodium sulfite solution was added, and the product was extracted with ethyl acetate. The solution was washed once each with de-ionized water and saturated aqueous sodium chloride solution. The organic layer was dried with anhydrous sodium sulfate, and the solvent was removed by distillation. Silica gel chromatography was subsequently used to purify the product, and a colorless, clear oil (Compound 14) (457 mg, 1.51 mmoles, 76%) was obtained. The analytical results for the Compound 14 (16-iodo-2,5,8,11,14-pentaoxahexadecane) obtained are shown below.
¹H-NMR (400 MHz, CDCl₃, TMS, rt) δ 3.76 (2H, t, J = 6.9 Hz), 3.66-3.64 (14H, m), 3.56-3.54 (2H, m), 3.38 (3H, s), 3.26 (2H, t, J = 6.9 Hz)

Compound 3 (25.5 mg, 0.0606 mmoles) obtained in Synthesis Example 1 was dissolved in 3.0 ml of methylene chloride, and Compound 14 (547 mg, 1.51 mmoles) described above was added in several portions. The reaction mixture was heated and refluxed for forty-four hours and was left standing to cool. The solvent was subsequently removed by distillation, and silica gel chromatography was used to purify the product to yield a dark red oil (Compound 12) (45.4 mg, 0.0580 mmoles, 96%). The analytical results for the Compound 12 (4-{5-[4-(dihexylamino) phenyl] thiophene-2-yl}-1-(2,5,8,11,14-pentaoxahexadecano-16-yl) pyridinium iodide) obtained are shown below. ¹H-NMR (400 MHz, CDCl₃, TMS, rt) δ 9.10 (2H, d, J = 6.8 Hz), 7.89 (2H, d, J = 6.9 Hz), 7.82 (1H, d, J = 4.1 Hz), 7.50 (2H, d, J = 8.8 Hz), 7.26-7.25 (1H, m), 6.63 (2H, d, J = 8.9 Hz), 4.94 (2H, t, J = 4.1 Hz), 4.04 (2H, t, J = 4.2 Hz), 3.66-3.49 (12H, m), 3.33-3.29 (7H, m), 1.60 (4H, brs), 1.33 (12H, brs), 0.91 (6H, t, J = 6.4 Hz)

Compound 3 (28.8 mg, 0.0684 mmoles) obtained in Synthesis Example 1 was dissolved in 0.50 ml of methylene chloride, and allyl iodide (Sigma-Aldrich Corporation) (500 µl, 5.48mmoles) was added. The reaction mixture was agitated for thirty minutes at 35°C and was left standing to cool. The solvent was subsequently removed by distillation, and silica gel chromatography was used to purify the product to yield dark red solids (Compound 16) (1.38 mg, 4.50 mmoles, 90%). The analytical results for the Compound 13 ({1-allyl-4-[5-(4-dihexylamino) phenyl] thiophene-2-yl} pyridinium iodide) obtained are shown below.
¹H-NMR (400 MHz, CDCl₃, TMS, rt) δ 8.89 (2H, d, J = 7.1 Hz), 7.89 (2H, d, J = 7.1 Hz), 7.81 (1H, d, J = 4.2 Hz), 7.51 (2H, d, J = 8.9 Hz), 7.27-7.23 (1H, m), 6.63 (2H, d, J = 9.0 Hz), 6.17-6.07 (1H, m), 5.63-5.54 (2H, m), 5.37 (2H, d, J = 6.4 Hz), 3.32 (4H, t, J = 7.7 Hz), 1.60 (4H, brs), 1.33 (12H, brs), 0.92 (6H, t, J = 6.8 Hz)

### Synthesis Example 4

In the present synthesis example, 1-allyl-4-{5-[4-(bis(2-hydroxyethyl)amino) phenyl] thiophene-2-yl} pyridinium iodide (Compound 15) containing polymer segments and with improved polar solvent solubility due to the hydroxyl groups introduced to the electron donating sections was synthesized. The synthesis route is shown in Figure 4.
2,2'-(Phenylimino) diethanol (Kanto Chemical Co., Ltd.) (905 mg. 5.00 mmoles) was dissolved in a mixed solvent of 30.0 ml of pyridine and 30.0 ml of dioxane. The solution was cooled with ice, and 3.81 g of iodine (Wako Pure Chemical Industries, Ltd.) (15.0 mmoles) was added. The solution was agitated for 1.5 hours at 0°C. The cooling ice bath was removed, and the solution was agitated for thirty minutes. A cooling ice bath was reapplied, and an aqueous sodium sulfite solution was added. The product was extracted with ethyl acetate and was washed once each with deionized water and saturated sodium chloride solution. The organic layer was subsequently dried using anhydrous sodium sulfate. The solvent was removed by distillation, and the product was purified using medium pressure fractionating liquid chromatography to yield white solids (Compound 16) (1.38 g, 4.50 mmoles, 90%). The analytical results for the Compound 16 [2,2'-(4-iodophenyl azanezyl) diethanol] obtained are shown below. ¹H-NMR (400 MHz, CDCl₃, TMS, rt) δ 7.48-7.44 (2H, m), 6.51-6.48 (2H, m), 3.86 (4H, t, J = 4.9 Hz), 3.57 (4H, t, J = 4.9 Hz), 2.77 (2H, m)

4-Bromopyridine hydrochloride salt (Wako Pure Chemical Industries, Ltd.) (150 mg, 0.773 mmoles), 2,5-thiophene diborate (Wako Pure Chemical Industries, Ltd.) (399 mg, 2.32 mmoles), sodium carbonate (Wako Pure Chemical Industries, Ltd.) (328 mg, 3.09 mmoles) and tetrakis (triphenylphosphine) palladium (0) (Wako Pure Chemical Industries, Ltd.) (26.9 mg, 0.0232 mmoles) were dissolved in a mixed solvent containing 5 ml of toluene and 5 ml of methanol. Air was removed to create vacuum and nitrogen was substituted. The [purging] process was repeated three times, and the reaction mixture was agitated for five hours at 70°C. After letting the reaction solution stand until cooled, the reaction solution was diluted with ethyl acetate and methylene chloride. The reaction solution was washed once with deionized water and once with saturated aqueous sodium chloride solution. The organic layer was dried using anhydrous sodium sulfate. The solvent was removed by distillation, and the product was purified using medium pressure fractionating liquid chromatography to obtain an orange powder (Compound 17) (25.2 mg, 0.0762 mmoles, 10%). The analytical results for the Compound 17 synthesized (2,2'-{4-[5-(pyridine-4-yl) thiophene-2-yl] phenyl azanediyl} diethanol) are shown below.
¹H-NMR (400 MHz, CDCl₃, TMS, rt) δ 8.47-8.46 (2H, m), 7.65-7.64 (3H, m), 7.53 (2H, d, J = 8.8 Hz), 7.25 (1H, d, J = 3.9 Hz), 6.80 (2H, d, J = 8.9 Hz), 3.76 (4H, t, J = 5.9 Hz), 3.60 (4H, t, J = 5.9 Hz)

Compound 17 (12.1 mg, 0.0356 mmoles) obtained in the manner described above was dissolved in 1.0 ml of methylene chloride, and allyl iodide (Sigma-Aldrich Corporation) (500 µl, 5.48 mmoles) was added. The reaction solution was heated and refluxed for eight hours. After the reaction solution was allowed to cool, the solvent and allyl iodide were removed by distillation to yield dark red solids (Compound 15) (17.2 mg, 0.0339 mmoles, 95%). The analytical results for the Compound 15 (1-allyl-4-{5-[4-(bis(2-hydroxyethyl) amino] phenyl} thiophene-2-yl) pyridinium iodide obtained are shown below.
¹H-NMR (400 MHz, CDCl₃, TMS, rt) δ 8.63 (2H, d, J = 7.0 Hz), 8.13 (2H, d, J = 7.0 Hz), 8.06 (1H, d, J = 4.1 Hz), 7.60 (2H, d, J = 8.8 Hz), 7.44 (2H, d, J = 4.2 Hz), 6.83 (2H, d, J = 8.9 Hz); 6.22-6.12 (1H, m), 5.54-5.48 (2H, m), 5.09 (2H, d, J = 6.2 Hz), 3.77 (4H, t, J = 5.9 Hz), 3.63 (4H, t, J = 5.9 Hz)

### Synthesis Example 5

In the present synthesis example, the 6 pyridine derivatives described below were newly synthesized in addition to Compounds 3 and 10 in order to investigate the effect of the alkyl chain length in R⁷ and R⁸ in the chemical formula 1.

**[Table 1]**

| Chemical formula 1 | | Compound number | |
|---|---|---|---|
| R7 | R8 | Dye of chemical formula 1 | Cation type dye of chemical formula 3(1) |
| Ethyl group | Ethyl group | 18 | |
| n-Butyl group | n-Butyl group | 19 | |
| n-Hexyl group | n-Hexyl group | 3 | |
| n-Octyl group | N-Octyl group | 20 | |

The same process used to synthesize Compound 3 was used with the exception of using 248 mg (0.9 mmoles) of N,N-diethyl-4-iodoaniline in place of N,N-dihexyl-4-iodoaniline, and 31 mg (34% yield) of yellow powder was obtained. The analytical results for the Compound 18 {N,N-diethyl-4-[5-(pyridine-4-yl) thiophene-2-yl] benzene amine} synthesized are shown below.
¹H-NMR (300 MHz, CDCl₃, TMS, rt) δ 8.57-8.55 (m, 2H), 7.49-7.43 (m, 5H), 7.15 (d, 1H, *J* = 3.8 Hz), 6.69 (d, 2H, *J* = 9.0 Hz), 3.50 -3.36 (m, 4H), 1.19 (t, 6H, *J* = 7.0 Hz); ¹³C-NMR (75 MHz, CDCl₃, TMS, rt) δ 150.6, 148.1, 142.1, 137.6, 127.5, 126.8, 122.0, 121.3, 119.6, 112.0, 44.8, 13.0; ESI-HRMS (m/z) calculated for C₁₉H₂₀N₂S: 308.1347; found: 309.1423 [M-H]⁺

The same process used to synthesize Compound 3 was used with the exception of using 298 mg (0.9 mmoles) of N,N-dibutyl-4-iodoaniline in place of N,N-dihexyl-4-iodoaniline, and 44 mg (40% yield) of yellow powder was obtained. The analytical results for the Compound 19 {N,N-dibutyl-4-[5-(pyridine-4-yl) thiophene-2-yl] benzene amine} synthesized are shown below.
¹H-NMR (300 MHz, CDCl₃, TMS, rt) δ 8.56-8.52 (m, 2H), 7.50-7.43 (m, 5H), 7.13 (d, 1H, *J* = 3.9 Hz), 6.64 (d, 2H, *J* = 8.9 Hz), 3.30 (t, 4H, *J* = 7.9 Hz), 1.65-1.53 (m, 4H), 1.44-1.29 (m, 4H), 0.97 (t, 6H, *J* = 7.3 Hz); ¹³C-NMR (75 MHz, CDCl₃, TMS, rt) δ 150.2, 147.8, 141.8, 137.2, 127.0, 126.4, 120.8, 120.5, 120.6, 119.2, 111.7, 50.8, 29.5, 20.

The same process used to synthesize Compound 10 was used with the exception of using 399 mg (0.9 mmoles) of N,N-dioctyl-4-iodoaniline in place of N,N-dihexyl-4-iodoaniline, and 94 mg (67% yield) of yellow powder was obtained. The analytical results for the Compound 20 {N,N-dioctyl-4-[5-(pyridine-4-yl) thiophene-2-yl] benzene amine} synthesized are shown below.
¹H-NMR (300 MHz, CDCl₃, TMS, rt) δ 8.58 (d, 2H, *J* = 9.0 Hz), 7.51□7.45 (m, 5H), 7.15 (d, 1H, *J* = 3.9 Hz), 6.66 (d, 2H, *J* = 9.0 Hz), 3.31 (t, 4H, *J* = 7.4 Hz), 1.61 (brs, 4H), 1.33-1.27 (m, 20H), 0.89 (t, 6H, *J* = 6.4 Hz); ¹³C-NMR (75 MHz, CDCl₃, TMS, rt) δ149.6, 148.2, 142.3, 136.9, 127.2, 126.7, 121.5, 120.6, 119.3, 111.7, 51.1, 31.9, 29.4, 27.2, 22.7, 14.2; ESI-HRMS (m/z) calculated for C₃₁H₄₄N₂S: 476.3225; found: 477.3301 [M-H]⁺

The same process used to synthesize Compound 10 was used with the exception of using 20 mg (0.06 mmoles) of Compound 18 in place of Compound 3, and an equal amount of red solids (Compound 21) was obtained. The analytical results for the Compound 21 { 4-[5-( 4-diethylamino) phenyl] thiophene-2-yl)-1-methyl pyridinium trifluoromethane sulfonate} synthesized are shown below.
¹H-NMR (300 MHz, CDCl₃, TMS, rt) δ 8.44 (d, 2H, *J* = 6.9 Hz), 7.74 (d, 2H, *J* = 7.0 Hz), 7.69 (d, 1H, *J* = 4.1 Hz), 7.42 (d, 2H, *J* = 8.9 Hz), 7.13 (d, 1H, *J* = 4.1 Hz), 6.60 (d, 2H, *J* = 8.9 Hz), 4.17 (s, 3H), 3.37 (q, 4H, *J* = 7.0 Hz), 1.18 (t, 6H, *J* = 7.0 Hz); ¹³C-NMR (75 MHz, CDCl₃, TMS, rt) δ 155.7, 149.2, 148.9, 144.6, 134.0, 133.9, 132.2, 128.1, 127.9, 123.5, 123.3, 121.3, 121.1, 119.5, 111.9, 44.9, 13.0, 12.9; ESI-HRMS (m/z) calculated for C₂₁H₂₃F₃N₂O₃S_{2:} 472.5436; found: 323.1577 [M-CF₃O₃S]⁺

The same process used to synthesize Compound 10 was used with the exception of using 103 mg (0.28 mmoles) of Compound 19 in place of Compound 3, and an equal amount of red solids (Compound 22) was obtained. The analytical results for the Compound 22 {4-[5-( 4-dibutylamino) phenyl] thiophene-2-yl)-1-methyl pyridinium trifluoromethane sulfonate} synthesized are shown below.
¹H-NMR (300 MHz, CDCl₃, TMS, rt) δ 8.48 (d, 2H, *J* = 6.4 Hz), 7.78 (d, 2H, *J* = 6.2 Hz), 7.72 (d, 1H, *J* = 4.1 Hz), 7.43 (d, 2H, *J* = 8.3 Hz), 7.16 (d, 1H, *J* = 3.6 Hz), 6.60 (d, 2H, *J* = 8.5 Hz), 4.21 (s, 3H), 3.29 (t, 4H, *J* = 7.4 Hz), 1.89-1.52 (m, 4H), 1.44-1.25 (m, 4H), 0.97 (t, 6H, *J* = 7.1 Hz) ¹³C-NMR (75 MHz, CDCl₃, TMS, rt) δ 160.0, 149.6, 149.0, 144.6, 134.0, 139.9, 132.2, 128.0, 127.9, 123.3, 123.2, 121.3, 121.1, 119.5, 111.9, 51.2, 29.8, 20.7, 14.3; ESI-HRMS (m/z) calculated for C₂₅H₃₁F₃N₂O₃S_{2:} 528.6504; found: 379.2207 [M-CF₃O₃S]⁺

The same process used to synthesize Compound 10 was used with the exception of using 140 mg (0.29 mmoles) of Compound 20 in place of Compound 3, and an equal amount of red solids (Compound 23) was obtained. The analytical results for the Compound 23 { 4-[5-(4-dioctylamino) phenyl] thiophene-2-yl)-1-methyl pyridinium trifluoromethane sulfonate} synthesized are shown below.
¹H-NMR (300 MHz, CDCl₃, TMS, rt) δ 8.51 (d, 2H, *J* = 7.0 Hz), 7.80 (d, 2H, *J* = 7.1 Hz), 7.74 (d, 1H, *J*= 4.2 Hz), 7.45 (d, 2H, *J*= 8.9 Hz), 7.18 (d, 1H, *J*= 4.2 Hz), 6.60 (d, 2H, *J*= 9.0 Hz), 4.23 (s, 3H), 3.29 (t, 4H, *J*= 7.3 Hz), 1.59 (brs, 4H), 1.33-1.28 (m, 20H), 0.89 (t, 6H, *J*= 6.5 Hz); ¹³C NMR (75 MHz, CDCl₃, TMSrt) δ 156.1, 149.6, 149.1, 144.7, 134.2, 134.1, 132.2, 128.1, 128.0, 123.9, 121.3, 121.2, 119.5, 111.9, 51.5, 32.2, 29.9, 29.7, 27.7, 27.5, 14.6; ESI-HRMS (m/z) calculated for C₃₃H₄₇F₃N₂O₃S_{2:} 640.8625; found: 491.3462 [M-CF₃O₃S]⁺

### Synthesis Example 6

The N,N-dihexyl-4-[5-(pyridine-4-yl) thiophene-2-yl] benzene amine (Compound 4) (31.5 mg, 0.0748 mmole) obtained in Synthesis Example 1 was dissolved in 1.0 ml of methylene chloride, and 1.5 ml of methyl iodide (Kanto Chemical Co., Ltd.) (24.2 mmoles) was added. The reaction mixture was heated for twenty-four hours and refluxed. The reaction mixture was left standing to cool after which the solvent and methyl iodide were subsequently removed by distillation. The product was purified using medium pressure fractionating liquid chromatography to yield dark red solids (Compound 24) (14.8 mg, 0.0263 mmoles, 35%). The analytical results for the Compound 24 (2-{5-[4-(dihexylamino) phenyl] thiophene-2-yl}-1-aminopyridinium iodide of the formula below are shown. 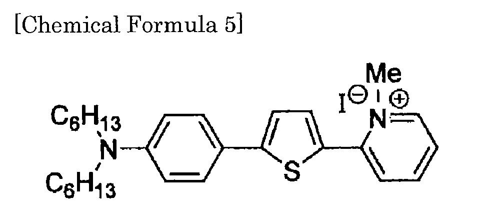 ¹H-NMR (400 MHz, CDCl₃, TMS, rt) δ 9.52 (1H, m), 8.40 (1H, m), 8.02 (1H,m), 7.92 (1H, m), 7.72 (1H, d, *J*= 4.0 Hz), 7.47 (2H, d, *J*= 8.9), 7.27 (1H, d, *J*= 4.0 Hz), 6.63 (2H, d, *J*= 8.9 Hz), 4.66 (3H, s), 3.30 (4H, m), 1.60 (4H, m), 1.32 (12H, m), 0.91 (6H, m).

### Example 1

In the present Example, the thiophene derivatives (Compounds 3 to 6) obtained in Synthesis Examples 1 and 2 were dissolved in a variety of solvents with different polarity, and the absorption and the fluorescent spectra were measured.
Now, the solvent polarity E_{T}(30) (kcal/mole) was obtained by dissolving the betaine dye of the following formula that is a light absorbing solvatochromic dye in each solvent and incorporating the maximum absorption wavelength, λ (nm), into the equation E_{T}(30) = 28591/λ. 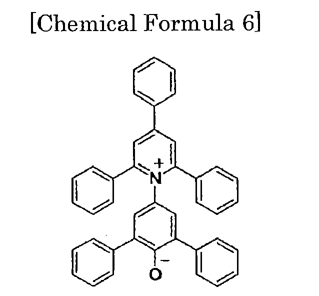 The thiophene derivative solutions were prepared by using a standard solvent for spectral measurement (Wako Pure Chemical Industries, Ltd.) in concentrations of from 0.7M to 1.2x10⁻⁵M, and absorptions were measured with a visible/ultraviolet spectrophotometer (V-560 UV/VIS Spectrophotometer manufactured by JASCO Corporation). The maximum absorption wavelength (nm) is shown in Table 2, and the molar absorption coefficient (mol⁻¹cm⁻¹) at the maximum absorption wavelength is shown in Table 3. The numbers in the tables indicate compound numbers.

**[Table 2]**

| Solvent | Solvent polarity E_{T} (30) | Maximum absorption wavelength (nm) | | | |
|---|---|---|---|---|---|
| | | No. 3 | No. 4 | No. 5 | No. 6 |
| 1,4-Dioxane | 36.0 | 389 | 387 | 395 | 490 |
| Acetone | 42.2 | 391 | 385 | 395 | 501 |
| DMSO | 45.1 | 401 | 395 | 406 | 497 |

**[Table 2]**

| Solvent | Solvent polarity E_{T} (30) | Molar absorption coefficient (mol⁻¹cm⁻¹) | | | |
|---|---|---|---|---|---|
| | | No. 3 | No. 4 | No. 5 | No. 6 |
| 1,4-Dioxane | 36.0 | 31,000 | 33,000 | 33,000 | 26,000 |
| Acetone | 42.2 | 33,000 | 33,000 | 33,000 | 36,000 |
| DMSO | 45.1 | 32,000 | 31,000 | 31,000 | 36,000 |

Next, fluorescence spectra of the thiophene derivatives were measured. The fluorescence spectra were measured using the same samples used for the absorption spectra and using a fluorescence spectrophotometer (F-4500 manufactured by Hitachi Ltd.)
The fluorescence emission maximum wavelength (nm) is shown in Table 4. The numbers in the table indicate compound numbers.

**[Table 4]**

| Solvent | Solvent polarity E_{T} (30) | Maximum fluorescence emission wavelength (nm) | | | |
|---|---|---|---|---|---|
| | | No. 3 | No. 4 | No. 5 | No. 6 |
| 1,4-Dioxane | 36.0 | 467 | 459 | 472 | 568 |
| Acetone | 42.2 | 507 | 488 | 507 | 639 |
| DMSO | 45.1 | 522 | 507 | 522 | 653 |

The experiment demonstrated that Compounds 3 to 6 (thiophene derivatives) have excellent solvatochromic fluorescence properties. Compound 6 with pyridinium as the electron donating group in particular had significantly longer absorption and emission wavelengths than Compounds 3 to 5. As a result, Compound 6 could be efficiently excited using commonly encountered argon lasers (488 nm).

### Example 2

In the present Example, Compound 6 was dissolved in aqueous synthetic detergent solutions having different pH values and the fluorescence was measured.
Compound 6 (0.1 g each) obtained in Synthesis Example 2 was placed in three test tubes, and 10 ml each of deionized water and one drop of weakly acidic "Biore" (liquid detergent of Kao Corporation), "COOP K Soft" (liquid detergent of Cope Green K.K.) or "My Pet" (liquid detergent of Kao Corporation) were added. Aqueous solutions of Compound 6 were obtained by dissolving the compound using ultrasonic irradiation. The solutions were irradiated with blue light (450 nm) using a full color LED flash light (MFL 143-FB manufactured by Anteya Technology Corporation). The fluorescent color observed was orange in a weakly acidic environment (pH = 5), red in a neutral environment (pH = 6) and crimson in a weakly alkaline environment (pH = 8). Compound 6 incorporated into detergent micelle was found to undergo an extensive change in its fluorescence wavelength in response to the electrical charges on the micelle surface.

### Example 3

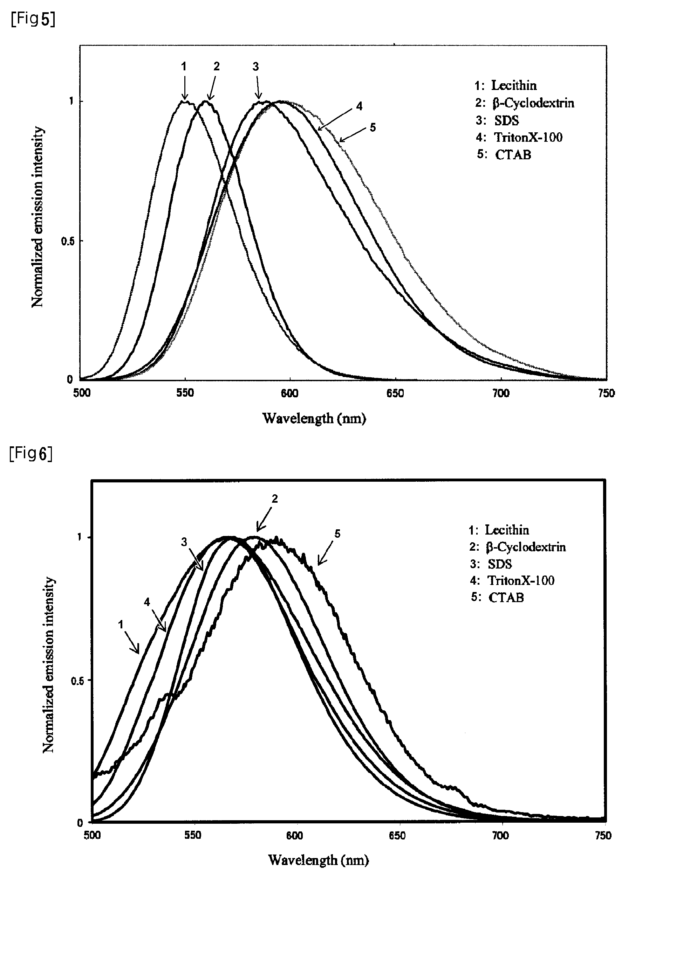

1 - 10 milligrams of egg yolk-derived phosphatidyl choline (lecithin), β-cyclodextrin, Triton X-100, cetyl trimethyl ammonium bromide (CTAB) and sodium dodecyl sulfate (SDS) were each added to 0.1 mg to 1 mg of Compound 10. After adding 5 milliliters of deionized water, the mixtures were sonicated with ultrasound for 20 seconds. The supernatant solution was irradiated with light at 488 nm, and the light emitted was measured using a fluorescence spectrophotometer (F-4500). The results are shown in Figure 5. Based on Figure 5, the dye (Compound 10) was found to respond sensitively to the local environment in terms of fluorescence wavelength.

### Example 4

1 - 10 milligrams of egg yolk-derived phosphatidyl choline (lecithin), β-cyclodextrin, Triton X-100, cetyl trimethyl ammonium bromide (CTAB) and sodium dodecyl sulfate (SDS) were each added to 0.1 mg to 1 mg of Compound 24. After adding 5 milliliters of deionized water, the mixtures were sonicated with ultrasound for 20 seconds. The supernatant solution was irradiated with light at 450 nm, and the light emitted was measured using a fluorescence spectrophotometer (F-4500). The results are shown in Figure 6.
Compound 24, like Compound 10, also responded sensitively to the local environment in terms of fluorescence wavelength. However, the fluorescence wavelength response, the emitted light wavelength change to the type of additives, of Compound 24 was smaller than that of Compound 10. In addition, the fluorescence intensity was less when the additive was CTAB.

### Example 5

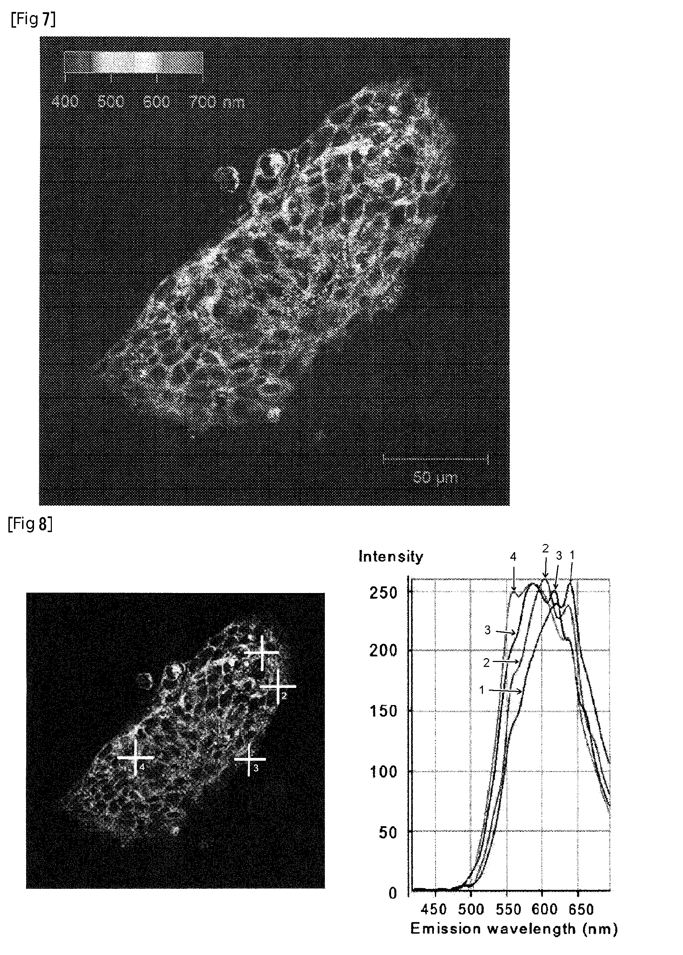

In the present Example, Compound 7 was introduced into cultured human colon cancer cell T84 and its image was observed using a fluorescence microscope.
Human colon cancer cell T84 culture (cell stock-microbial stock-gene bank (ATCC) number CCL-248, supplied by Summit Pharmaceuticals International Corporation) in a sub-confluent state that had been cultured (37°C, 5% CO₂ for two to four days in a glass bottom dish (35 mm, Matsunami Glass Ind.,Ltd.) was washed with phosphoric acid buffered physiological saline solution. One hundred microliters of a DMSO solution of 2x10⁻⁵ M of Compound 7 was added dropwise near the cells, and a reaction was allowed to occur for five minutes at room temperature. The cells were washed again using phosphoric acid buffered physiological saline solution and were immediately exposed to an argon laser (488 nm) to reach an excited state in the phosphoric acid buffered physiological saline solution. The response was observed using a confocal laser microscope (LSM510META manufactured by Carl Zeiss). The fluorescence microscope images obtained are shown in Figure 7.
As clearly indicated by the data in Figure 7, the cell membranes of colon cancer cells T84 are stained by Compound 7. As shown in Figure 8, fluorescence at various wavelengths from 560 nm to 640 nm was observed depending on the environment around each segment. Compound 7 could clearly be used as a probe capable of detecting subtle environmental changes measured by fluorescence wavelength. The cells showing fluorescence maintained the fluorescence over at least several hours when standing at room temperature. In addition, Compound 7 did not undergo laser caused fading when the compound was examined using a suitable confocal laser microscope under the conditions used in the experiment.

### Example 6

In the present Example, macrophage cells were stained using the pyridinium derivatives shown in Table 1 and the differences in cell staining were compared in order to investigate the effects of the alkyl chain lengths in R⁷ and R⁸.
Several micrograms of each pyridinium derivative (Compounds 10 and 21 to 23) were added to 10 milliliters of a liquid medium obtained by mixing calf serum and D-MEM base medium in a 1:9 ratio. The mixtures were left standing in a refrigerator overnight to dissolve the solids. 5 milliliters of the supernatant solutions were removed the next day and were used as cell staining solutions after filtering the solutions through sterilizing membrane filters with a pore diameter of 0.2 µm.
The macrophage cells (RAW264 of ATCC Co., Ltd.) were floated in HydroCell dishes manufactured by Cell Seed Co., Ltd. using a liquid culture obtained by mixing calf serum and D-MEM base medium in a 1:9 ratio.
The media were removed from the glass bottom dishes on which the RAW264 cells were supported, and 0.2 ml of each stain solution was added. The cells were stained by leaving the dish standing for fifteen minutes in a CO₂ incubator at 37°C. The stain solutions were subsequently removed, and the media were exchanged three times using a liquid medium obtained by mixing calf serum and D-MEM base medium in 1:9 ratio. The stained cells were exposed to an argon laser (488 nm) using a confocal laser microscope (LSM510 META manufactured by Carl Zeiss Co., Ltd.), and the fluorescence was observed in a random mode. The results are shown in terms of fluorescence microscopic images (Figure 9).
Entire cells were stained with fluorescence when the cells were stained using Compound 21, (1), and the identification of cell membrane and micro organs inside the cells was difficult. When Compounds 22 and 10 were used to stain, (2) and (3), the cell membrane and mitochondria were selectively stained and could be clearly identified by the difference in the fluorescence wavelength. In comparison, when Compound 23 was used to stain, (4), the entire cell was stained with fluorescence but the fluorescence intensity was low and the stain was not even.
Based on the results, the cell segments could be identified with high sensitivity particularly when the number of carbon atoms in the alkyl chains R⁷ and R⁸ were 3 to 7 in the chemical formula (Chemical Formula 1).

### Example 7

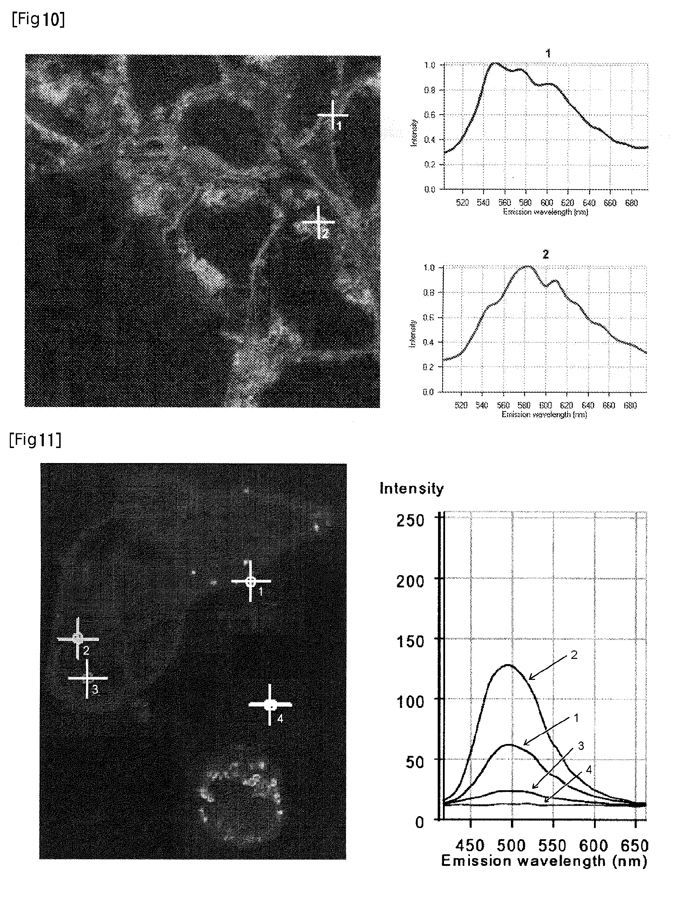

In the present Example, human fetus kidney-derived HEK293 cells were stained using Compound 10, and the location of the dye and emitted light wavelength were examined.
Several micrograms of Compound 10 was added to 10 milliliters of a liquid medium obtained by mixing calf serum and D-MEM base medium in a 1:9 ratio. The mixture was left standing in a refrigerator overnight to dissolve the solids. 5 milliliters of the supernatant solution was removed the next day and was used as a cell staining solution after filtering the solution through a sterilizing membrane filter with a pore diameter of 0.2 µm.
The human fetus kidney-derived cells HEK293 (Riken Cell Bank) were cultured in a five milliliter culture flask using a liquid culture obtained by mixing calf serum and D-MEM base medium in a 1:9 ratio. The solution was used to inoculate a glass bottom dish.
The medium was removed from the glass bottom dish on which the HEK293 cells were supported, and 0.2 ml of the stain solution was added. The cells were stained by leaving the dish standing for fifteen minutes in a CO₂ incubator at 37°C. The stain solution was subsequently removed, and the medium was exchanged three times using a liquid medium obtained by mixing calf serum and D-MEM base medium in a 1:9 ratio. The stained cells were exposed to an argon laser (488 nm) using a confocal laser microscope (LSM510 META manufactured by Carl Zeiss Co., Ltd.), and the fluorescence was observed in a random mode.
The results are shown in Figure 10. The emitted light color is different for the cell membrane (1 in the figure) and mitochondria (2 in the figure).
The results were interpreted to mean that mitochondria concentrations vary with the cell type and can be identified by the emission intensity.

### Comparative Example 1

In the present Comparative Example, the compound shown below described in Reference 3 (Japanese Patent Application Public Disclosure No. 2008-291210) was used in place of Compound 7, and the fluorescence microscope images of the cultured human colon cancer cell T84 were examined in the manner described in Example 5.

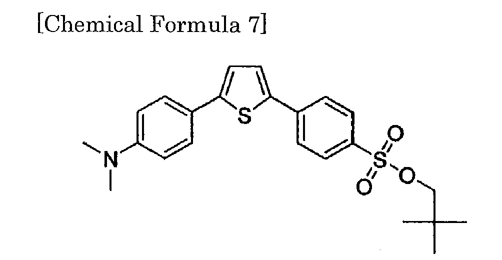

The fluorescence microscope images obtained and their fluorescence spectra are shown in Figure 11. In the case of the present comparative example, fluorescence image could be obtained only when excited by using a blue diode laser (405 nm) that is not mounted on ordinarily used fluorescence microscopes. And the cell membrane was selectively labeled with fluorescence as shown in the left figure of Figure 11, but the fluorescence wavelength remained almost unchanged for cell sections.

## Claims

1. A fluorescent solvatochromic dye represented by the following formula. 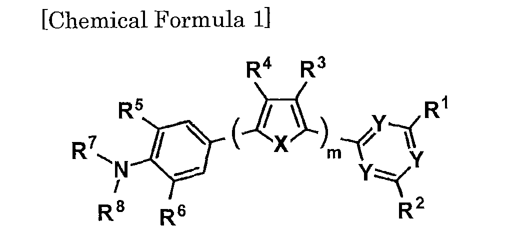 wherein,
X represents an oxygen atom (-O-) or a sulfur atom (-S-), m represents an integer from 1 to 4,
Y are independent each other and at least one of Y represents -N= with the remainder representing -CR⁹=,
R⁹ represents a hydrogen atom, an alkyl group with 1 to 4 carbon atoms or a group capable of bonding to other molecules,
R¹ and R² are independently hydrogen atoms, halogen atoms, primary or secondary amino groups or alkyl groups with 1 to 4 carbon atoms,
R³ and R⁴, which may be identical to or different from the other, are hydrogen atoms, alkoxy groups, acylamino groups, alkyl groups, halogen substituted alkyl groups, amino groups, hydroxyl groups or halogen atoms, and R³ and R⁴ may form, by sharing the carbon atoms bonding to R³ or R⁴, an aromatic or aliphatic 5, 6 or 8 membered ring that may contain ether bonds,
R⁵ and R⁶, which may be identical to or different from the other, are hydrogen atoms or alkyl groups,
R⁷ and R⁸, which may be identical to or different from the other, are hydrogen atoms or alkyl groups that may contain substituents, and R⁵ and R⁷ may form an aromatic or aliphatic 5 or 6 membered ring by sharing carbon atoms and nitrogen atoms bonding to R⁷ or R⁸, and R⁶ and R⁸ may form an aromatic or aliphatic 5 or 6 membered ring by sharing carbon atoms and nitrogen atoms bonding to R⁶ or R⁸.

2. The fluorescent solvatochromic dye of claim 1 wherein Y is independent and at least one of Y represents -N⁽⁺⁾R¹⁰=Z⁽⁻⁾ with the remainder representing -CR⁹= , wherein R⁹ is similarly defined as described above, R¹⁰ represents a hydrogen atom, a hydrocarbon group, a group capable of bonding with other molecules, or a hydrocarbon group having a group capable of bonding with other molecules and Z represents a monovalent anionic species.

3. The fluorescent solvatochromic dye of claim 1 or 2 wherein R¹ to R⁶ are hydrogen atoms.

4. The fluorescent solvatochromic dye described in any one of claims 1 to 3 wherein R⁷ and R⁸, which may be identical to or different from the other, are alkyl groups containing 3 to 7 carbon atoms.

5. A cell staining agent comprising the fluorescent solvatochromic dye of claim 2.

6. A cell staining solution comprising the fluorescent solvatochromic dye of claim 2 dissolved or dispersed in an aqueous solvent.

7. A cell staining solution comprising the fluorescent solvatochromic dye of claim 2 dissolved or dispersed in serum.

8. A cell stained with the fluorescent solvatochromic dye of claim 2.
